# EUROPEAN PATENT APPLICATION

(11) **EP 3 366 757 A1**
(43) Date of publication of application: **29.08.2018**
(21) Application number: 16857400.2
(22) Date of filing: 17.10.2016
(51) Int. Cl.: C12M 1/00

(54) **BUBBLE EJECTION CHIP, LOCAL ABLATION DEVICE, LOCAL ABLATION METHOD, INJECTION DEVICE, AND INJECTION METHOD**

(30) Priority: 19.10.2015 JP 2015205948
(71) Applicant: Bex Co., Ltd., Tokyo 173-0004 (JP)
(72) Inventor: YAMANISHI Yoko, Tokyo 135-8548 (JP); KAMBAYASHI Takuya, Tokyo 135-8548 (JP); TAKAHASHI Kazuki, Tokyo 135-8548 (JP)
(74) Representative: Heyerhoff Geiger & Partner Patentanwälte PartGmbB
(86) International application number: PCT/JP2016/080692
(87) International publication number: WO 2017/069085

(57) **Abstract**

A bubble-jetting chip capable of jetting bubbles upward from a substrate is provided.

Bubbles can be jetted upward from a substrate by a bubble-jetting chip comprising:
at least a substrate, an energizing portion, and a bubble-jetting portion;
the energizing portion being formed on the substrate;
the bubble-jetting portion comprising an electrode that is formed of a conductive material, a shell part formed of an insulating photosensitive resin, and an extended section that extends from the shell part, the shell part covering a periphery of the electrode, the extended section extending beyond a tip of the electrode, and the bubble-jetting portion further comprising a space formed between the extended section and the electrode; and
the electrode of the bubble-jetting portion being formed on the energizing portion.

## Description

### BACKGROUND OF THE INVENTION

### 1. Field of the Invention

The present invention relates to a bubble-jetting chip, a local ablation device and local ablation method, and an injection device and injection method. The invention particularly relates to a bubble-jetting chip in which a bubble-jetting portion is formed facing upward on a flat surface of a substrate so that an electrode is connected to an energizing portion formed on the substrate, whereby a bubble-jetting outlet is oriented upward and bubbles can be jetted upward when the bubble-jetting chip is placed, as well as a local ablation device, local ablation method, injection device, and injection method that include the bubble-jetting chip.

### 2. Description of the Related Art

Along with recent developments in biotechnology, there has been an increased demand for local processing of cells, etc., such as opening holes in cell membranes or walls, and removal of nuclei from cells or introducing DNA and other nucleic acid materials into cells. There are widely known methods of local processing techniques (referred to below as "local ablation methods"), including contact processing techniques using electric scalpels and other probes and non-contact ablation techniques using lasers, etc. In particular, most recently there has been devised a technique for contact processing with an electric scalpel, in which a sintered surface is kept to an order of several micrometers whereby a thermal invasion area is contained and resolution is improved (see Non-Patent Document 1).

With laser processing, there have been dramatic advances made with femtosecond lasers, and most recently there has been devised a technique for performing cellular processing (see Non-Patent Document 2) or a laser processing technique in which the production of bubbles is suppressed in a liquid phase.

However, the prior-art contact processing techniques using electric scalpels and other probes have had the characteristic of burning through the object due to the Joule heat generated by continuous high-frequency waves, and accordingly a problem has been presented in regard to the prominent effect of thermal invasion into peripheral tissues due to roughness of the cutting surface and heat. Also, in the non-contact processing techniques using femtosecond lasers and other lasers, a problem has been presented in regard to the effect of thermal invasion of tissues surrounding the cutting surface by local impact of high-density energy.

Meanwhile, there are widely known local physical injection techniques (referred to below as "injection methods") for introducing nucleic acid materials, etc., into cells, etc., including electroporation, sonoporation using ultrasonic waves, and particle gun methods.

However, in prior-art electroporation techniques, there has been a limit to improvements in penetrability of cell membranes by electric field intensity, complications are presented in regard to injection into objects having hard cell membranes or cell walls rather than soft lipid bilayers, and other complications have arisen in regard to injection into locally targeted areas due to restrictions on arrangement of electrodes, etc. Also, in sonoporation using ultrasonic waves, focusing of ultrasonic waves has been difficult, local cavitation of bubbles occurs, and resolution is not readily increased. With injection methods using particle gun methods, problems have been presented in that materials attached to the particle surfaces detach from the surfaces during particle injection and the introduction rate has been low. With electroporation, sonoporation, and particle gun methods, problems are encountered in regard to the high consumption of injected materials and difficulty related to injection of precious materials.

In order to solve the problems of the prior-art local ablation methods and injections described above, the present inventors discovered that cutting (local ablation) of a processed object can be performed by: producing a bubble-jetting member comprising a core that is formed of a conductive material, a shell part that is formed of an insulating material, covers the core, and includes a section extending from the tip of the core, and a space that is formed between the extended section of the shell part and the tip of the core; immersing the bubble-jetting member in a solution; applying a high-frequency voltage to the solution to produce bubbles; and continuously releasing the bubbles onto the processed object. An application for patent was thus filed (see Patent Document 1).

The inventors also discovered that bubbles in which a solution of dissolved and/or dispersed injection material is adsorbed on the interfaces thereof can be produced by providing an outer shell part on the outside of the shell part of the bubble-jetting member so as to leave a space with the shell part, and introducing a solution of dissolved and/or dispersed injection material into the space; and a processed object can be cut and the injection material contained in the solution covering the bubbles can be injected into the processed object by continuously releasing the bubbles onto the processed object. An application for patent was thus filed (see Patent Document 1).

### Prior Art Documents

### Patent Documents

[Patent Document 1] Japanese Patent 5526345

### Non-Patent Documents

[non-Patent Document 1] D. Palanker et al., J. Cataract. Surgery, 38, 127-132, (2010)
[non-Patent Document 2] T. Kaji et al., Applied Physics Letters, 91, 023904, (2007)

### SUMMARY OF THE INVENTION

### Problem to Be Solved by the Invention

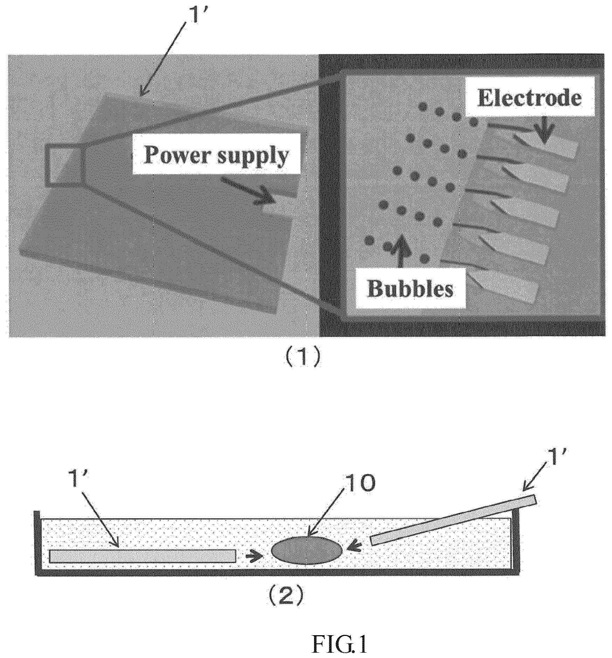

The present inventors have developed a bubble-jetting chip (sometimes referred to hereinafter as a "multi-bubble-jetting portion chip 1'") in which a bubble-jetting portion is formed by providing an electrode between two photosensitive resins layered on a substrate, and an upper face of the bubble-jetting chip is packaged in PDMS in order to further prevent leakage of electricity (Yoko YAMANISHI et al., "Multi-bubble-jetting portion Injection Using Microarray Electrode," No. 15-2 Proceedings of the 2015 JSME Conference on Robotics and Mechatronics, Kyoto, Japan, May 17-19, 2015, referred to below as "non-patent document 3"). The bubble-jetting portion shown in FIG. 1(1) is formed to be parallel with the substrate. Therefore, when the multi-bubble-jetting portion chip 1' shown in FIG. 1(1) is used upon being immersed in a solution in a Petri dish, etc., because the multi-bubble-jetting portion chip 1' is a substrate assuming the form of a thin plate, usage methods are common in which the multi-bubble-jetting portion chip 1' is immersed into the Petri dish, etc., from a horizontal or inclined direction, as shown in FIG. 1(2), and not from a vertical direction. As shall be apparent, it is possible to immerse the multi-bubble-jetting portion chip 1' in a vertical direction, but in this case, the depth of the Petri dish, etc., must be greater than the size of the multi-bubble-jetting portion chip 1'.

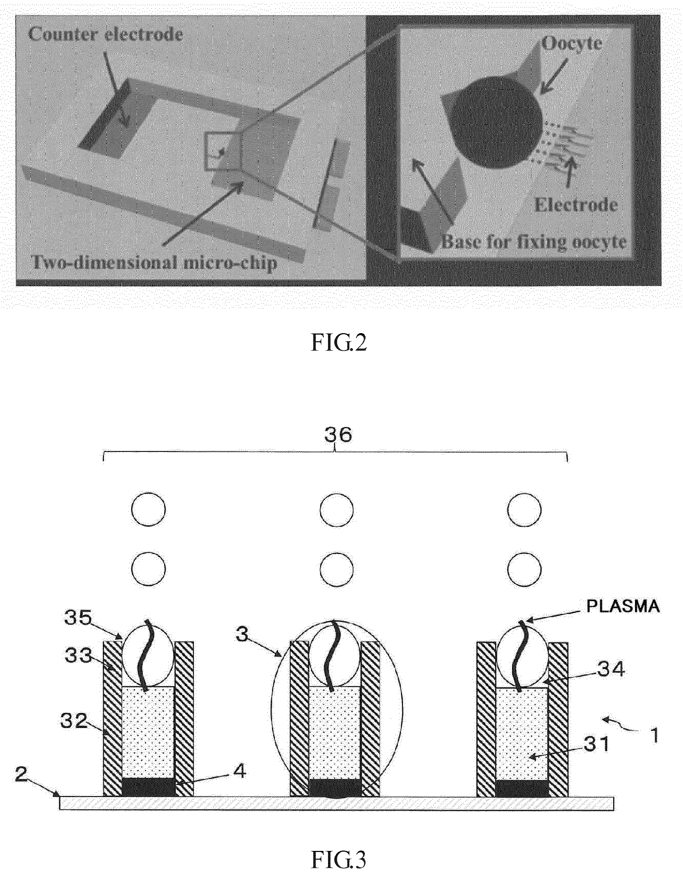

Non-patent document 3 also indicates that the location where the processed object is gripped is formed in front of the bubble-jetting portion, as shown in FIG. 2, but the location where the processed object shown in FIG. 2 is gripped is part of a configuration intended for bubbles to be jetted horizontally.

The present application is an invention contrived in order to solve the abovementioned problems. After thoroughgoing research, it was newly discovered that:
(1) by photolithographically forming an energizing portion on a substrate and forming a bubble-jetting portion oriented upward so that an electrode is connected to the energizing portion formed on the substrate, a bubble-jetting outlet could be oriented upward when a bubble-jetting chip is placed on a Petri dish, etc., and bubbles could be jetted in a direction substantially perpendicular to the flat surface of the substrate; and
(2) by forming a photosensitive resin constituting the bubble-jetting portion into a cylindrical shape by etching from above the substrate, a bubble-jetting chip on which a desired number of bubble-jetting portions are disposed at desired positions could be fabricated.

Specifically, a purpose of the present application is to provide a bubble-jetting chip, a local ablation device and local ablation method, and an injection device and injection method.

### Means for Solving the Problems

The present application relates to the bubble-jetting chip, the local ablation device and local ablation method, and the injection device and injection method presented below.

(1) A bubble-jetting chip, comprising:
   at least a substrate, an energizing portion, and a bubble-jetting portion;
   the energizing portion being formed on the substrate;
   the bubble-jetting portion comprising an electrode that is formed of a conductive material, a shell part that is formed of an insulating photosensitive resin, and an extended section that extends from the shell part, the shell part covering a periphery of the electrode, the extended section extending beyond a tip of the electrode, and the bubble-jetting portion further comprising a space formed between the extended section and the electrode; and
   the electrode of the bubble-jetting portion being formed on the energizing portion.
(2) The bubble-jetting chip according to (1) above, wherein two or more of the bubble-jetting portions are formed.
(3) The bubble-jetting chip according to (2) above, wherein the heights of the bubble-jetting portions are different.
(4) The bubble-jetting chip according to any of (1) to (3) above, wherein the photosensitive resin is a negative photoresist.
(5) The bubble-jetting chip according to any of (1) to (4) above, wherein a counter electrode that constitutes an electrode pair with the electrode of the bubble-jetting portion is formed on the substrate.
(6) The bubble-jetting chip according to any of (1) to (5), further comprising an outer shell part formed around the bubble-jetting portion, and a space between the bubble-jetting portion and the outer shell part being capable of storing a solution containing an injection material.
(7) The bubble-jetting chip according to (6) above, wherein a channel for delivering a solution containing the injection material and/or a solution for forming an assisting flow is formed in the space.
(8) A local ablation device, comprising the bubble-jetting chip according to any of (1) to (7) above.
(9) An injection device, comprising the bubble-jetting chip according to any of (1) to (7) above.
(10) A local ablation method, wherein:
   at least the space of the bubble-jetting chip of the local ablation device according to claim 8 is filled with a solution;
   high-frequency pulses are applied to an electrode pair configured with the electrode of the local ablation device and the counter electrode to cause bubbles to be released from the tip of the bubble-jetting portion; and
   a processed object is processed with the bubbles.
(11) The local ablation method according to (10) above, wherein electricity is discharged from the electrode when the high-frequency pulses are applied.
(12) An injection method, wherein:
   at least the space of the bubble-jetting chip of the injection device according to (9) above is filled with a solution containing an injection material;
   high-frequency pulses are applied to an electrode pair configured with the electrode of the injection device and the counter electrode to cause the release of bubbles onto which the solution containing the injection material is adsorbed; and
   the injection material is introduced into the processed object while local ablation is performed on the processed object with the bubbles.
(13) The injection method according to (12) above, wherein electricity is discharged from the electrode when the high-frequency pulses are applied.

### Effects of the Invention

In the bubble-jetting chip of the present application, a bubble-jetting portion is formed so that a bubble-jetting outlet opens upward from a substrate. Therefore, bubbles can be jetted upward in a solution; therefore, when the bubbles advance in the solution, there will be little change in the advancing direction due to buoyancy. Also, the bubbles do not adhere to, *inter alia,* members constituting the bubble-jetting chip 1. Furthermore, because the bubble-jetting portion faces upward when the bubble-jetting chip is disposed on the bottom part of a Petri dish or another container, local ablation or local injection can be performed on a processed object from below.

### BRIEF DESCRIPTION OF THE DRAWINGS

FIGS. 1(1) and (2) illustrate a scheme of the multi-bubble-jetting portion chip 1';
FIG. 2 illustrates a scheme of the multi-bubble-jetting portion chip 1';
FIG. 3 illustrates a scheme of an example of an embodiment of the bubble-jetting chip 1;
FIGS. 4(1) to (4) illustrate another embodiment of the bubble-jetting chip 1;
FIG. 5-1 illustrates an example of the manufacturing steps of the bubble-jetting chip 1;
FIG. 5-2 illustrates an example of the manufacturing steps of the bubble-jetting chip 1;
FIG. 5-3 illustrates an example of the manufacturing steps of the bubble-jetting chip 1;
FIGS. 6(1) to (4) illustrate another embodiment of the bubble-jetting chip 1;
FIG. 7 illustrates the overall configuration of an example of an embodiment of a local ablation device 6 using the bubble-jetting chip 1;
FIG. 8 illustrates a scheme of an example of an embodiment of the bubble-jetting chip 1 applied to an injection device;
FIG. 9 is a cross-sectional view along line A-A' of FIG. 8;
FIGS. 10(1) to (6) illustrate an example of an embodiment in which the bubble-jetting chip 1 is used in needle-less infusion;
FIG. 11(1) is a photograph used in lieu of a drawing, and is a photograph of the bubble-jetting chip 1 produced in example 1; FIG. 11(2) is a photograph used in lieu of a drawing, and is a photograph showing the vicinity of the bubble-jetting portion 3 enlarged; FIG. 11(3) is a drawing for representing the dimensions of the vicinity of the produced bubble-jetting portion 3; FIG. 11(4) is a photograph used in lieu of a drawing, and is a photograph in which the bubble-jetting portion 3 and at least part of a counter electrode 5 are disposed inside a frame 41;
FIG. 12 is a photograph used in lieu of a drawing, and is a photograph of the generation of bubbles 36 captured with a high-speed camera; and
FIG. 13 is a photograph used in lieu of a drawing, and is a photograph showing electrical discharge from an electrode 31 in addition to the jetting of bubbles in example 3.

### DESCRIPTION OF THE PREFERRED EMBODIMENTS

The bubble-jetting chip, local ablation device and local ablation method, and injection device and injection method are described in detail below with reference to the accompanying drawings.

FIG. 3 illustrates a scheme of an example of an embodiment of the bubble-jetting chip 1. The bubble-jetting chip 1 includes at least a substrate 2, a bubble-jetting portion 3, and an energizing portion 4. The energizing portion 4 is formed on the substrate 2. Also, the bubble-jetting portion 3 includes an electrode 31 that is formed of a conductive material, a shell part 32, an extended section 33, and a space 34. More specifically, the shell part 32 covers the periphery of the electrode 31, and the extended section 33 extends from the shell part 32 beyond the tip of the electrode 31. The space 34 is formed between the extended section 33 and the tip of the electrode 31, and a bubble-jetting outlet 35 is formed on the side opposite from the electrode 31. The space 34, aside from the bubble-jetting outlet 35, is covered by the electrode 31 and the extended section 33, as shown in FIG. 3. In other words, a space (channel) for feeding a liquid, etc., is not formed between the electrode 31 and the shell part 32; the space 34 communicates with the exterior via the bubble-jetting outlet 35 alone. By applying a voltage to the electrode 31 and the counter electrode 5, which is not illustrated in FIG. 3, the bubbles 36 can be continuously jetted. As shall be described hereinafter, increasing the voltage applied to the electrode 31 makes it possible to discharge electricity from the electrode 31 as well as jet the bubbles 36. Also, as shall be described hereinafter, the counter electrode 5 may be formed on the substrate 2 or may be separate from the bubble-jetting chip 1.

The material for forming the substrate 2 is not particularly limited provided that the energizing portion 4 can be layered thereon. Examples include glass, quartz, PMMA, and silicon.

The material for forming the electrode 31 is not particularly limited provided that the material can be energized and can be deposited on the energizing portion 4 by electroplating, electroless plating, or other methods. Examples include nickel, gold, platinum, silver, copper, tin, magnesium, chromium, tungsten, and other metals, or alloys thereof.

In the bubble-jetting chip 1, the shell part 32 and the extended section 33 are made by using photolithography. Accordingly, the material for forming the shell part 32 and the extended section 33 is not particularly limited provided that the material is an insulating photosensitive resin. Examples include commercial TSMR V50, PMER, and other positive photoresists, and SU-8, KMPR, and other negative photoresists. Because bubbles 36 are jetted by energizing the electrode 31 and the counter electrode 5, a load is easily applied to the bubble-jetting outlet 35, which is a very small portion, particularly when high voltage is applied thereto. Because SU-8, KMPR, and other negative photoresists have higher hardness than positive photoresists, a negative photoresist is preferably used as the photosensitive resin when high voltage is applied to the bubble-jetting portion 3.

The material of the energizing portion 4 and the counter electrode 5 is not particularly limited provided that electricity can be delivered from an external power supply to the electrode 31, and the same material as that of the abovementioned electrode 31 can be used. When the counter electrode 5 is produced separately, the counter electrode should be capable of being energized with the electrode 31 and therefore is not particularly limited to being in the form of a rod, sheet, or other shape. When the counter electrode 5 is formed on the substrate 2, the counter electrode should be layered on the substrate 2 in the same manner as the energizing portion 4.

Because bubbles formed in the space 34 are jetted from the bubble-jetting outlet 35 so as to be dispersed violently when electricity is outputted to the electrode 31 and the counter electrode 5, there is no need to supply air from the outside to the bubble-jetting portion 3.

Also, when bubbles 36 are formed inside the space 34, bubbles having a size near the inner diameter (indicated as "diameter D" or "D" below) of the bubble-jetting outlet 35 are produced. Accordingly, the depth (length from the tip of the electrode 31 to the bubble-jetting outlet 35; indicated as "L" below) of the space 34 must be large enough for bubbles to be produced inside the space 34, and the L/D ratio is preferably at least 1. Meanwhile, the upper limit of the L/D ratio is not particularly limited provided that the size is sufficient for the bubbles 36 to be continuously jetted. The L/D ratio can be adjusted according to the thickness of the layered photosensitive resin and the height of the deposited electrode 31. The size of the jetted bubbles 36 can be adjusted by changing the diameter D of the bubble-jetting outlet 35, and should be adjusted by the shape of the photomask during production.

In the embodiment of the bubble-jetting chip 1 illustrated in FIG. 3, an example in which a plurality of bubble-jetting portions 3 are formed is shown in regard to the described relationship, but it is also an option to have only one bubble-jetting portion 3. Also, as is depicted in the manufacturing method described hereinafter, a bubble-jetting chip in which a desired number of bubble-jetting portions 3 are arranged at desired positions can be made by forming the shell part 32 and the extended section 33 constituting the bubble-jetting portion 3 by etching into cylinders from above on the substrate 2 in the bubble-jetting chip 1.

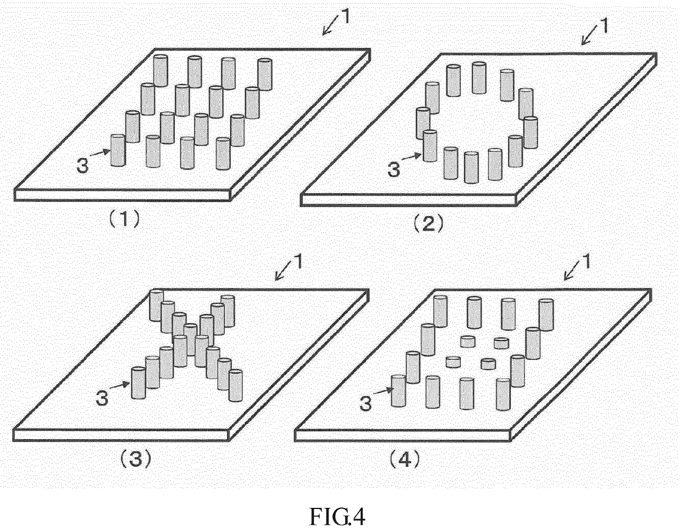

FIG. 4 illustrates another embodiment of the bubble-jetting chip 1. For example, the bubble-jetting portions 3 can be formed in a desired number and a desired arrangement, such as the equal spacing shown in FIG. 4(1), the circle shown in FIG. 4(2), and the X shape shown in FIG. 4(3). In addition to arranging the bubble-jetting portions 3 in a desired number at desired positions, the heights of the bubble-jetting portions 3 can be changed as shown in, e.g., FIG. 4(4) by repeating exposure and etching while changing the thickness of the photosensitive resin layering and the shape of the photomask. There are cases in which, when injection is performed on the processed object, injection is performed not in a single location, but all at once on a plurality of locations on the processed object in a linear formation, a planar formation, etc. When the multi-bubble-jetting portion chip 1' is used, injection can be performed in a planar formation, in addition to a linear formation, by layering a plurality of multi-bubble-jetting portion chips 1'. However, when a plurality of multi-bubble-jetting portion chips 1' are layered, the bubble-jetting outlets can be formed in a planar formation, but it is difficult to set the arrangement of bubble-jetting outlets as desired in, e.g., a circular shape, an X shape, etc. In the bubble-jetting chip 1 of the present application, the desired number of bubble-jetting portions can be arranged at the desired positions. Furthermore, when the heights of the bubble-jetting portions 3 are changed as shown in FIG. 4(4), the distance between the bubble-jetting outlets and the processed object can be kept constant even if the processed object has a three-dimensional shape.

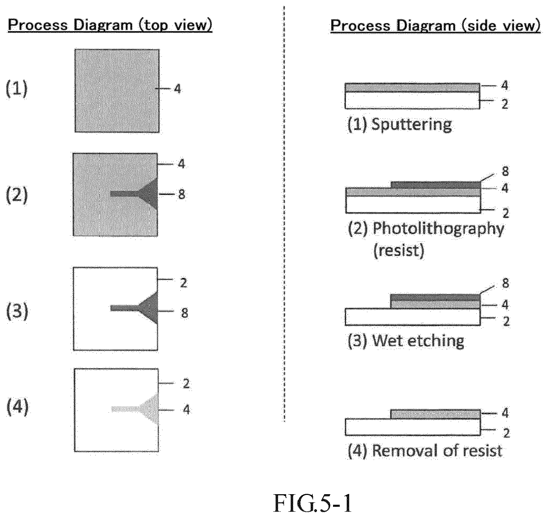

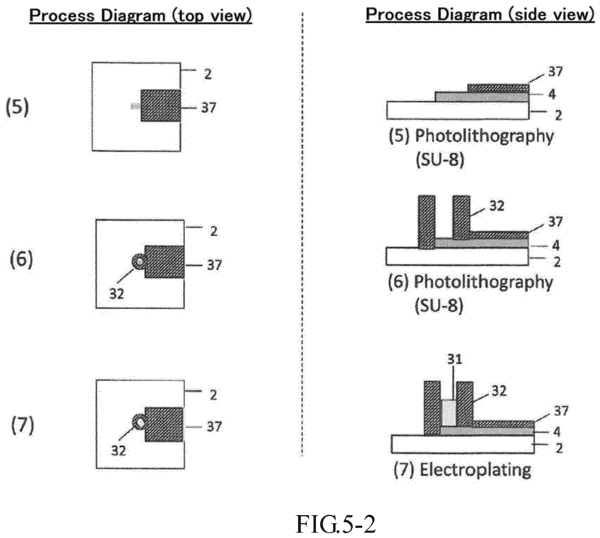

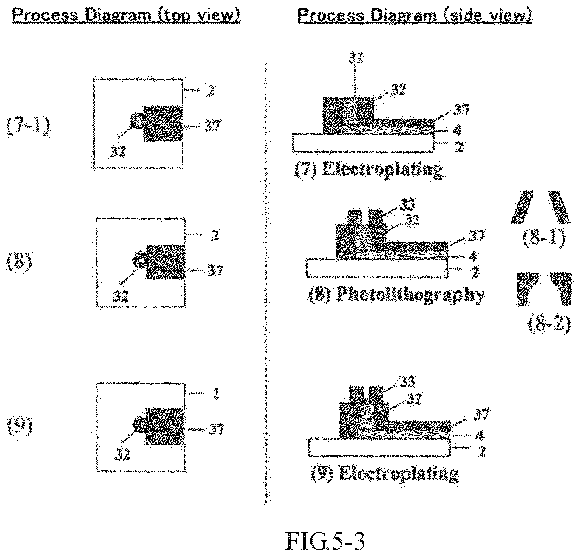

FIGS. 5-1 to 5-3 illustrate one example of the manufacturing steps of the bubble-jetting chip 1 of the present application. In FIGS. 5-1 to 5-3, an example in which there is one bubble-jetting portion 3 is shown in regard to the depicted relationship, but the shape of the photomask may be changed when a plurality of bubble-jetting portions 3 is formed.
(1) The material for forming the energizing portion 4 is layered on the substrate 2 by sputtering.
(2) A photoresist 8 is applied, and photoexposure and development are performed using a mask so that the photoresist 8 remains in the portion where the energizing portion 4 is ultimately to be formed.
(3) The material other than the portion where the energizing portion 4 is to be formed is removed by wet etching or another method.
(4) The photoresist 8 is removed, whereby the energizing portion 4 is formed. Though not illustrated, when the counter electrode 5 is formed on the substrate 2, the counter electrode 5 should be formed simultaneously with the energizing portion 4 by changing the shape of the photomask.
(5) A photoresist is applied, and photoexposure and development are performed using a mask so that the photoresist remains on an unnecessary portion of the energizing portion 4 (the portion where the bubble-jetting portion 3 is not formed). Because the photoresist is cured by photoexposure and development, an insulating layer 37 is formed in the following steps without removing the photoresist.
(6) A photoresist is applied, and photoexposure and development are performed using a photomask designed in a configuration such that the shell part 32 and the extended section 33 remain. The photoresist cured by photoexposure and development becomes the shell part 32 and the extended section 33. The size of the electrode 31 of the bubble-jetting portion 3 should adjust the size of the photomask.
(7) The bubble-jetting chip 1 shown in this embodiment can be made by growing the electrode 31 via electroplating on the energizing portion 4. The bubble-jetting chip 1 differs from the multi-bubble-jetting portion chip 1' shown in FIGS. 1 and 2 in that the entire periphery of the electrode 31 is covered by the cylindrical photosensitive resin (shell part 32, extended section 33) as shown in (6) and (7). The bubble-jetting outlet 35 can then be made to face upward from the substrate 2 by growing the electrode 31 on the energizing portion 4. The term "upward from the substrate" signifies either a substantially vertical direction relative to the plane of the substrate 2 on which the energizing portion 4 is formed, or the direction in which the energizing portion 4 is layered as seen from the substrate 2. Though these two expressions are different, they have the same meaning. Accordingly, the bubble-jetting outlet 35 can be arranged facing upward in a Petri dish, etc., and the bubbles 36 can be jetted upward by immersing the bubble-jetting chip 1 in a Petri dish, etc., with the surface without the bubble-jetting portion 3 placed on the lower side. Therefore, when the bubbles 36 advance in the solution, there will be less change in the advancing direction due to buoyancy, while at the same time, because the jetting direction and advancing direction of the bubbles 36 are the same, the bubbles do not accumulate in the periphery of the bubble-jetting outlet 35. Because the electrode 31 should have continuity with the counter electrode 5 in the bubble-jetting chip 1, for example, a frame including the bubble-jetting portion 3 and at least part of the counter electrode 5 may be formed on the bubble-jetting chip 1, and the frame may be filled with a conductive solution.

The bubble-jetting chip 1 may be made in various forms by repeating photoresist application, exposure, and development. For example:
(7-1) Instead of the procedure of (7) described above, the electrode 31 is grown to the same height as the shell part 32.
(8) A photoresist is applied, and photoexposure and development are performed using a photomask designed to a shape such that the extended section 33 remains. In (8), there is a risk of electricity leakage when the electrode 31 is exposed. Therefore, the extended section 33 should be formed so as to span over the shell part 32 and the electrode 31. When the outside diameter of the extended section 33 is less than the outside diameter of the electrode 31, the exposed electrode 31 should be masked by a photoresist, PDMS, etc., after the extended section 33 is formed. Also, while the extended section 33 is made by photolithography in the procedure described above, the extended section 33 may be formed separately by three-dimensional molding or a processing technique and may be adhered with an adhesive, etc. A tapered shape depicted in (8-1) and (8-2) is given as an example for a separately made extended section 33, but other shapes may also be used. As described above, the size, speed, invasiveness, directionality, and other attributes of the bubbles can be adjusted by making the extended section 33 smaller than the shell part 32.
(9) After the procedure of (8) described above, an upper electrode 31 may be grown by electroplating as needed. In the case that (9) is carried out, the size, speed, invasiveness, directionality, and other attributes of the bubbles can be adjusted by adjusting the height of the electrodes.

In the manufacturing method described above, because the thickness and inside diameter of the cylindrical photosensitive resin can be set as desired, the extended section 33 is not easily damaged even if the applied voltage is increased. Therefore, the electrodes can be enlarged, and as a result, electricity can be discharged from the electrodes in addition to the jetting of the bubbles by raising the voltage applied to the electrodes. Accordingly, electric discharge processing can be carried out on the processed object in addition to local ablation or injection by bubbles, and the present invention can therefore be used as a device for treating cancer, etc. Also, holes can be opened in plant cells and other biomaterial, as well as a broad range of hard materials such as metals, polymers, etc., and genes, reagents, etc. can also be introduced as needed.

In the procedure shown in FIGS. 5-1 to 5-3, the heights of the bubble-jetting portions 3 are all the same. When the heights of the bubble-jetting portions 3 are changed as shown in FIG. 4(4), procedure (6) should be repeated after the procedure of (6) described above, in which case the height of the applied photoresist should be different from the height of the shell part 32 depicted in (6).

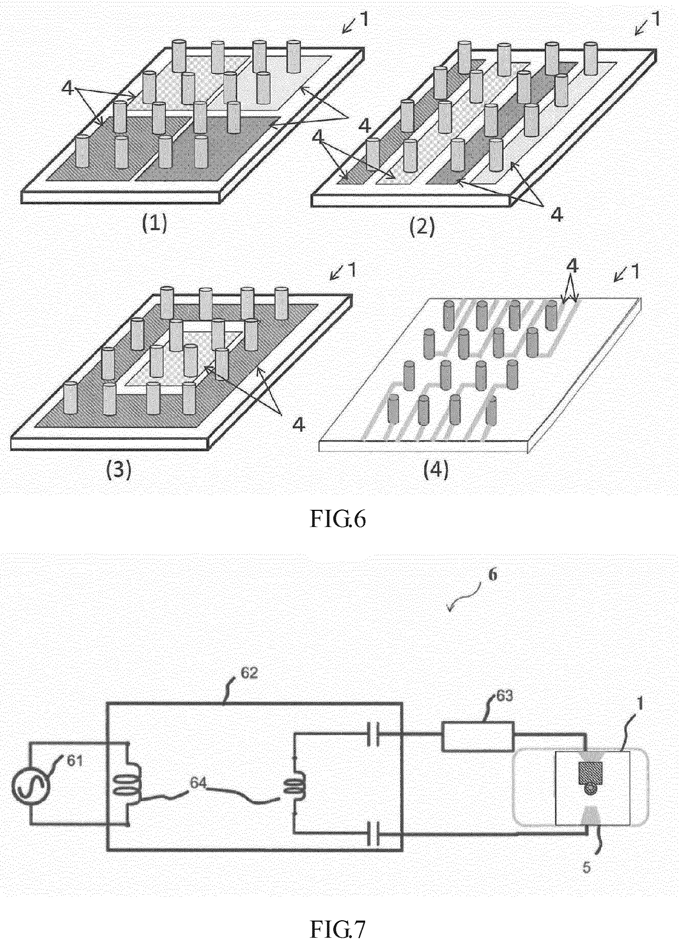

FIG. 6 illustrates another example of an embodiment of the bubble-jetting chip 1. When a plurality of bubble-jetting portions 3 are formed, all of the bubble-jetting portions 3 may be formed on a single energizing portion 4, but the energizing portion 4 may be divided and formed as a plurality. FIG. 6(1) illustrates an example in which the energizing portion 4 is divided into block units, FIG. 6(2) an example in which the energizing portion 4 is divided into line units, and FIG. 6(3) an example in which the energizing portion 4 is divided into an inside portion and an outside portion, but there are no particular limitations as to the divided units. Due to the dividing of the energizing portion 4, the voltage applied to the bubble-jetting portions 3 can be varied among the divided units when local ablation or local injection is performed. For example, when a spherical processed object is subjected to local ablation or local injection using the bubble-jetting chip 1 shown in FIG. 6(3), any discrepancy in the strength of the bubbles coming into contact with the processed object can be minimized by having the voltage of the outside portion, where the distance between the bubble-jetting outlets and the processed object is greater, be higher than the voltage of the inside portion. Also, the energizing portion 4 need not be divided into block units; an energizing portion 4 may be provided for each bubble-jetting portion 3 as shown in FIG. 6(4). The format shown in FIG. 6(4) can be used in cell patterning etc. When voltage is applied all at once to a plurality of bubble-jetting portions 3 provided on one energizing portion 4, a high voltage must be applied to the energizing portion 4. In the embodiment of FIG. 6(4), the electricity output means can be made smaller because voltage should be applied to the individual bubble-jetting portions 3.

The resists, etchants, sputtering devices, etc., used in the abovementioned steps may be publicly known reagents and devices used in the field of micromachining technology.

FIG. 7 illustrates the overall configuration of an example of an embodiment of a local ablation device 6 using the bubble-jetting chip 1. The local ablation device 6 shown in FIG. 7 includes electricity output means. The electricity output means include at least a generic commercial AC power supply device 61, and an electric wire 62 for forming a circuit between the electrode 31 of the bubble-jetting chip 1 and the counter electrode 5, and may also have a non-dielectric resistor 63, a voltage amplification circuit 64, a digital input/output (DIO; not shown) port, etc., as needed. The electricity output means can be fabricated merely by incorporating a non-dielectric resistor 63, DIO port, etc. in a prior-art electrical circuit for an electric scalpel, and setting to an output configuration for use on microscopic objects.

The current, voltage, and frequency of the electricity output to the electrode 31 and the counter electrode 5 are not particularly limited provided that the ranges are such that bubbles can be jetted, electricity can be discharged from the electrode 31 as needed, and the bubble-jetting portion 3 is not damaged. For example, the current is preferably 10 mA to 10 A, and more preferably 25 mA to 800 mA. It is undesirable for the current to be less than 10 mA, since it may not be possible to properly produce bubbles 36, or for the current to be greater than 10 A, since wear of the electrode may occur. The voltage is preferably 100 V to 100 kV, and more preferably 200 V to 8.0 kV. It is undesirable for the voltage to be smaller than 100 V, since production of bubbles 36 may be difficult, or for the voltage to be greater than 100 kV, since wear of the electrode 31 or damage to the extended section 33 might occur. The frequency is preferably 1 kHz to 1 GHz, more preferably 5 kHz to 1 MHz, and particularly preferably 10 kHz to 60 kHz. It is undesirable for the frequency to be less than 1 kHz, since the extended section 33 might be damaged, or for the frequency to be greater than 1 GHz, since it might not be possible to produce bubbles 36. The numerical values given above are standard numerical values and can be changed depending on the size of the electrode 31.

In the local ablation method, first, the bubble-jetting chip 1 of the local ablation device 6 and the counter electrode 5 are immersed in a conductive solution. A processed object is arranged on the bubble-jetting portion 3 of the bubble-jetting chip 1, and bubbles 36 jetted from the bubble-jetting portion 3 are caused to collide with the processed object, whereby local ablation of the processed object can be performed.

The processed object is not particularly limited provided that ablation can be performed thereon using bubbles. Examples include cells and proteins. Examples of cells include stem cells isolated from human or non-human animal tissues, skin cells, mucous cells, liver cells, islet cells, nerve cells, cartilage cells, endothelial cells, epithelial cells, bone cells, muscle cells, egg cells, and other animal cells, and plant cells, insect cells, *E. coli,* yeast, molds, and other microbial cells, and other cells. When electricity is discharged from the electrode 31, the effects of electric discharge processing are also achieved in addition to those of processing by bubbles; therefore, a harder processed object may be used. Examples include rice, plant cells, and other comparatively hard biological samples, as well as resins, metals, etc. "Processing" in the present application signifies jetting bubbles on a processed object, as well as performing electric discharge as needed, to open holes in the object or cut a portion of the object.

In Patent Document 1, the present inventors demonstrated that bubbles jetted from the bubble-jetting member could adsorb an injection material. Presumably, the bubbles produced by energizing the core are charged with electricity, and the injection material is adsorbed onto the bubbles due to the electricity. Accordingly, when performing local ablation using the bubble-jetting chip 1 illustrated in FIG. 3 or FIG. 4, if an injection material is caused to be contained in the conductive solution in which the bubble-jetting chip 1 is immersed, bubbles 36 around which the injection material is adsorbed can be jetted. Therefore, the injection material can be introduced while performing local ablation on the processed object.

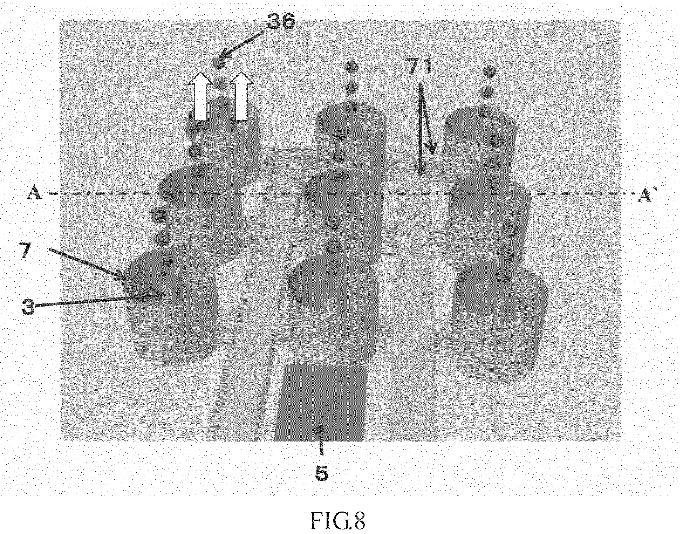

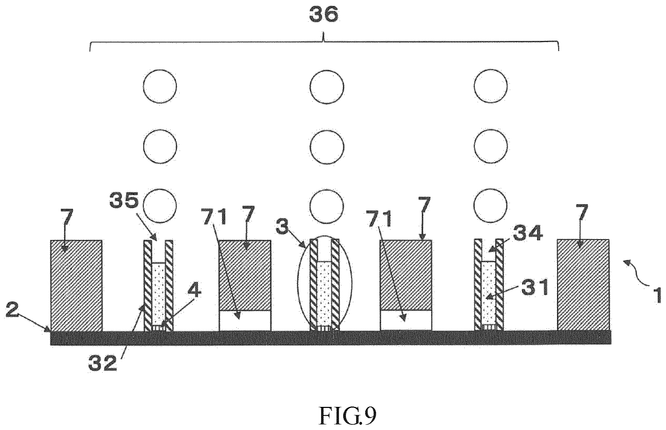

FIG. 8 illustrates a scheme of an example of an embodiment of the bubble-jetting chip 1 (referred to below as "bubble-jetting chip for injection") applied to an injection device. FIG. 9 is a cross-sectional view along line A-A' of FIG. 8. In the bubble-jetting chip 1 shown in FIGS. 8 and 9, an outer shell part 7, which can be filled with a solution containing an injection material, is formed in on the outer periphery of the bubble-jetting portion 3. The space between the outer shell part 7 and the bubble-jetting portion 3 is filled with a solution containing a first injection material, whereby the first injection material can be adsorbed to the peripheries of the bubbles 36 jetted from the bubble-jetting outlet 35. The outer shell part 7 is formed separately from the bubble-jetting chip 1 and should be disposed around the bubble-jetting portion 3.

A channel 71 for sending a conductive solution as needed may be formed so as to be connected to the outer shell part 7. A solution containing the first injection material can be sent through the channel 71 to the space between the outer shell part 7 and the bubble-jetting portion 3.

When the channel 71 is formed, it is permissible to continue to send a conductive liquid that does not contain the first injection material. In such a case, the conductive liquid forms a liquid flow in the direction in which the bubble-jetting portion 3 extends, as shown by the white arrows in FIG. 8. This liquid flow acts as an assisting flow to assist the movement of the bubbles 36, whereby the force of the bubbles 36 coming into contact with the processed object can be increased.

The outer shell part 7 and the channel 71 can be fabricated by photolithography, nanoimprinting, etching, three-dimensional molding, three-dimensional processing, UV curing, stereolithography, two-photon absorption photo-molding, etc. Also, the material for fabricating the outer shell part 7 and the channel 71 is preferably an insulating material, and should be polydimethylsiloxane (PDMS), parylene, epoxy resin, polyimide, polyethylene, glass, quartz, PMMA, silicon, or another well-known insulating material.

An injection device can be produced by using the bubble-jetting chip 1 for injection instead of the bubble-jetting chip 1 of the local ablation device 6 mentioned above. Except for filling the space between the outer shell part 7 and the bubble-jetting portion 3 with a solution containing an injection material, the same procedure as the local ablation method can be used to introduce the injection material while performing local ablation on a processed object.

The injection material is not particularly limited, whether gas, solid, or liquid, provided that the material can be dissolved and/or dispersed in a liquid. Examples of gases include air, nitrogen, helium, carbon dioxide, carbon monoxide, argon, and oxygen; examples of solids include DNA, RNA, proteins, amino acids, and inorganic substances; and examples of liquids include chemical solutions and amino acid solutions. Examples of solutions for dissolving and/or dispersing the injection materials include physiological saline and culture media.

When the outer shell part 7 shown in FIGS. 8 and 9 is disposed on the periphery of the bubble-jetting portion 3, the solution filled into the space between the bubble-jetting portion 3 and the outer shell part 7 can be held by surface tension. Accordingly, the bubble-jetting chip 1 for injection shown in FIGS. 8 and 9 can be used to perform local ablation or injection into the ambient air; i.e., for needle-less infusion. When used for needle-less infusion, the bubble-jetting chip 1 shown in FIGS. 8 and 9 should be brought into direct contact with the processed object in the ambient air.

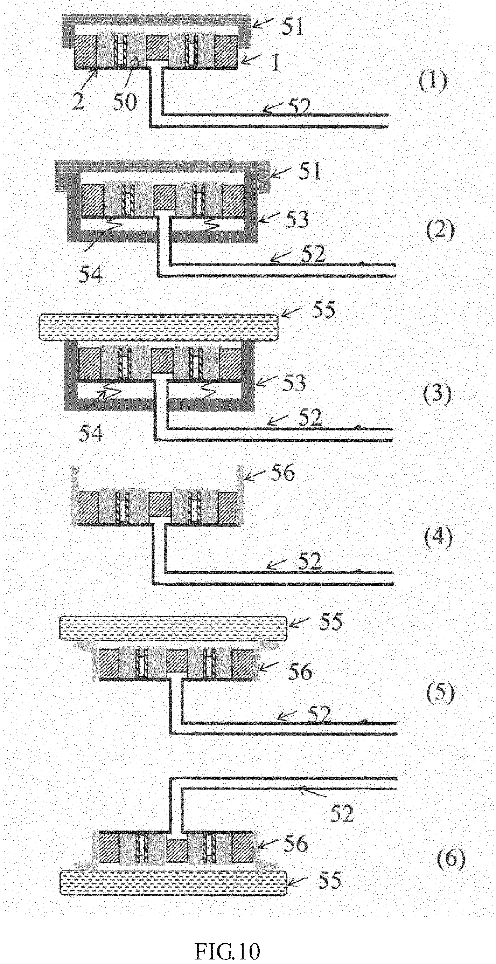

FIGS. 10(1) to (6) illustrate an example of an embodiment in which the bubble-jetting chip 1 is used in needle-less infusion. When needle-less infusion is maintained in a solution 50 (sometimes referred to below as a "filling solution") being filled from above into the space between the bubble-jetting portion 3 and the outer shell part 7, the filling solution 50 must be prevented from drying. FIG. 10(1) shows an example in which a cap 51 is provided to the periphery of the bubble-jetting chip 1. Also, a support 52 may be attached to the substrate 2 of the bubble-jetting chip 1 in order to facilitate bringing the bubble-jetting chip 1 into contact with the processed object. The support 52 may be fabricated into a solid configuration, but it is also an acceptable option to make the support 52 have a hollow configuration and pass through the interior thereof an electric wire for applying voltage to the energizing portion 4 and the counter electrode 5. Also, when the support 52 is a hollow shape, a channel may be formed in the interior thereof and connected with the channel 71 shown in FIG. 8, whereby the filling solution can be continuously supplied. In this case, the cap 51 is not needed. The same applies to the embodiments below.

FIGS. 10(2) and (3) are drawings for depicting another embodiment of needle-less infusion. When needle-less infusion is used, the structure is preferably one that allows the distance between the bubble-jetting outlet and the processed object to be finely adjusted. FIGS. 10(2) and (3) illustrate an embodiment in which fine adjustment of this distance is made easier. In the present embodiment, a movable frame 53 that can slide relative to the bubble-jetting chip 1 is provided to the periphery of the bubble-jetting chip 1, the support 52 is slidably inserted through a hole formed in the movable frame 53, and the support 52 is fixed to the bubble-jetting chip 1. Also, a spring or other urging means 54 is provided between the movable frame 53 and the substrate 2 of the bubble-jetting chip 1, and the movable frame 53 is designed to protrude beyond the bubble-jetting chip 1. When the needle-less infusion of the present embodiment is used, the cap 51 is taken off, the tip of the movable frame 53 is brought into contact with the processed object 10, and the support 52 is pushed in the opposite direction from the urging force of the spring, whereby the distance between the bubble-jetting outlet and the processed object 10 can be finely adjusted.

FIGS. 10(4) and (5) are drawings for describing another embodiment of needle-less infusion. In the embodiment shown in FIGS. 10(4) and (5), instead of the movable frame 53, a variable frame 56 is formed from silicon or another flexible material around the bubble-jetting chip 1. The tip of the variable frame 56 is designed to protrude beyond the bubble-jetting chip 1 before use, as shown in FIG. 10(4). When the needle-less infusion of the present embodiment is used, the cap is removed and the tip of the variable frame 56 is brought into contact with and pushed into the processed object 10, whereby the variable frame 56 deforms and the distance between the bubble-jetting outlet and the processed object 10 can be finely adjusted.

The formats shown in FIGS. 10(1) to (5) described above are mere illustrations of cases in which the bubble-jetting chip 1 is used for needle-less infusion; other embodiments may also be adopted. Also, FIGS. 10(1) to (5) illustrate examples in which needle-less infusion is used from below the processed object, but because the filling solution 50 has surface tension, needle-less infusion may also be used with the bubble-jetting outlet oriented downward as shown in FIG. 10(6).

The embodiments are described specifically below with examples, but these examples are provided simply for reference to specific modes for description of the embodiments of the present application. These illustrations do not represent restrictions or limitations on the scope of the present invention disclosed in the present application.

### Examples

### <Example 1>

### [Production of bubble-jetting chip 1]

(1) Au was formed into a film on a glass substrate using a sputtering device (Vacuum Device MSP-30T) with plasma current value (80 mA) for one minute.
(2) OFPR-800 LB (200CP) was spun-coated on the glass substrate at 2000 rpm for 30 seconds and 7000 rpm for 2 seconds, and the coated substrate was pre-baked in an oven at 90°C for 30 minutes. Next, photoexposure was performed using a chrome mask, and development was performed using NMD-3. After development, the resulting product was rinsed with ultrapure water and dried upon the water being cast off in a spin dryer, etc.
(3) The areas other than the patterned OFPR were soaked with an Au etchant (AURUM-302, Kanto Chemical) to etch the Au, and the resulting product was rinsed with ultrapure water.
(4) The glass substrate was immersed in acetone and the remaining OFPR film was removed, with which patterning of the Au electrode portion was completed, as was the counter electrode 5.
(5) SU-8 3005 was spun-coated at 2000 rpm for 30 seconds on the glass substrate, and the coated substrate was pre-baked on a hot plate at 95°C for 3 minutes. Photoexposure was performed using a chrome mask, and then the resulting product was post-exposure baked on a hot plate at 95°C for 3 minutes. Development was performed using PGMEA (2-Methoxy-1-methylethyl acetate; CAS Number: 142300-82-1), and the water was cast off in a spin dryer to allow drying, with which a SU-8 insulating layer was produced.
(6) SU-8 3050 was spun-coated at 1000 rpm for 30 seconds and at 4000 rpm for two seconds on the glass substrate, and the coated substrate was pre-baked on a hot plate at 95°C for 50 minutes. Next, photoexposure was performed using a chrome mask, and post-exposure baking was performed on the hot plate at 95°C for five minutes. Lastly, development was performed using PGMEA (2-Methoxy-1-methylethyl acetate; CAS Number: 142300-82-1), and the water was cast off in a spin dryer to allow drying, with which a shell part 32, a cylindrical structure of SU-8, was produced.
(7) An electrode was connected to the Au patterned part, and Ni plating was grown to the height (100 pm) of the SU-8 pattern along the inner side of the SU-8 shell part 32.
(8) SU-8 3050 was spun-coated at 800 rpm for 30 seconds and at 4000 rpm for two seconds on the glass substrate, and the coated substrate was pre-baked on a hot plate at 95°C for 50 minutes. Next, photoexposure was performed using a chrome mask, and post-exposure baking was performed on the hot plate at 95°C for 5 minutes. Lastly, development was performed using PGMEA (2-Methoxy-1-methylethyl acetate; CAS Number: 142300-82-1), and the water was cast off in a spin dryer to allow drying, with which a cylindrical structure of SU-8 (a shell part 32 and an extended section 33) was produced over the shell part 32 produced in (6).
(9) An electrode was connected to the Au patterned part, and Ni plating was grown along the inner side of the SU-8 cylindrical structure to 30 µm from the top of the SU-8 cylindrical structure.

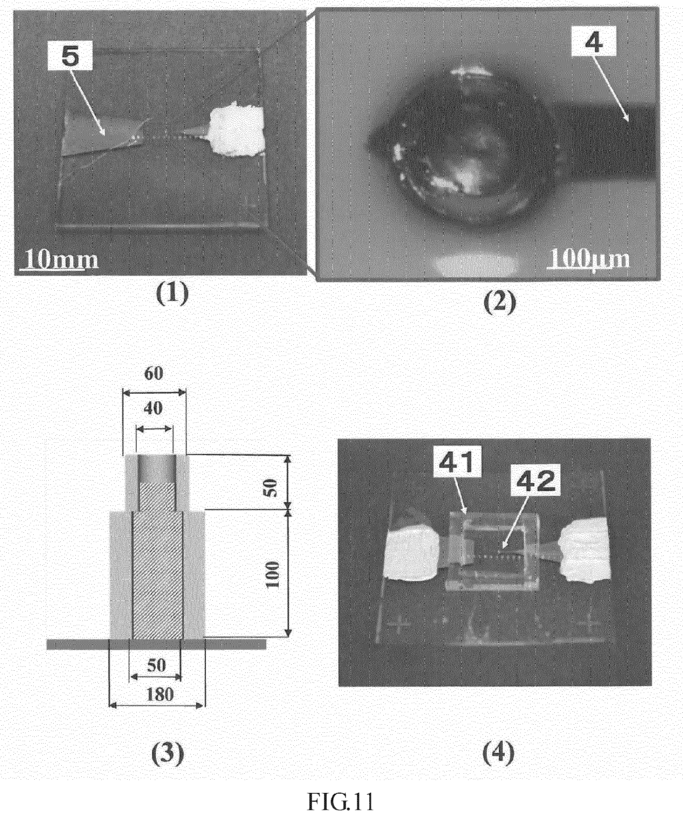

FIG. 11(1) is a photograph of the bubble-jetting chip 1 produced in example 1, and FIG. 11(2) is a photograph enlarging the vicinity of the bubble-jetting portion 3. Also, FIG. 11(3) is a drawing for representing the dimensions of the vicinity of the produced bubble-jetting portion 3. The thickness of the lower shell part 32 was about 65 pm, the diameter of the electrode 31 was about 50 pm, the height of the bubble-jetting portion 3 was about 150 pm, and the diameter of the bubble-jetting outlet was about 40 pm.

### <Example 2>

### [Production of local ablation device and injection device and bubble jetting experiment]

The bubble-jetting chip 1 produced in example 1 was incorporated in place of the scalpel of an electric scalpel for medical use (product of ConMed Corp., Hyfrecator 2000), a non-dielectric resistor and a DIO port were furthermore incorporated in the electricity output means, and a local ablation device and injection device were thus produced.

Next, PDMS was used to produce a frame 41 large enough for the bubble-jetting portion 3 and at least part of the counter electrode 5 to be disposed therein, and the frame 41 was affixed to the bubble-jetting chip 1, as shown in FIG. 11(4). The inside of the frame 41 was filled with a 2.5M NaCl solution 42. Electricity was outputted to the electrode 31 and the counter electrode 5 with a current of 27.7 mA, a voltage of 309 V, an output frequency of 450 kHz, a sampling frequency for impedance matching of 450 kHz, and feedback at 3.5 kHz. The formation of bubbles was captured using a high-speed camera (VW-9000, product of Keyence Corp.) from the side face of the frame 41.

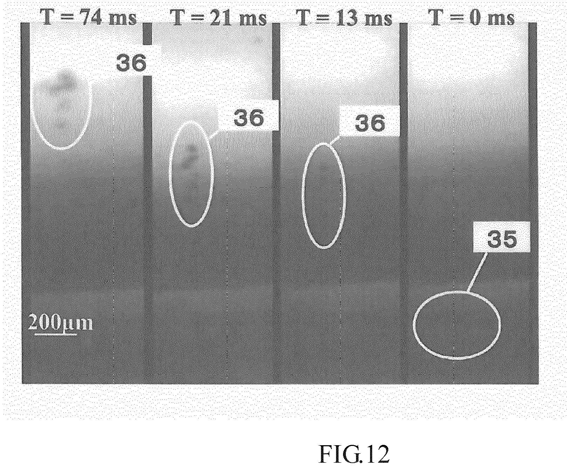

FIG. 12 is a photograph of the generation of bubbles 36 captured by a high-speed camera. As is clear from the photograph, it was confirmed that bubbles 36 could be jetted upward from the bubble-jetting outlet 35 by using the bubble-jetting chip 1 produced in example 1. Also, bubble accumulation in the periphery of the bubble-jetting outlet was not observed. Presumably, this is because the bubble-jetting outlet faced upward and the bubbles moved smoothly upward due to buoyancy in addition to bubble jetting force.

### <Example 3>

### [Jetting of bubbles containing plasma]

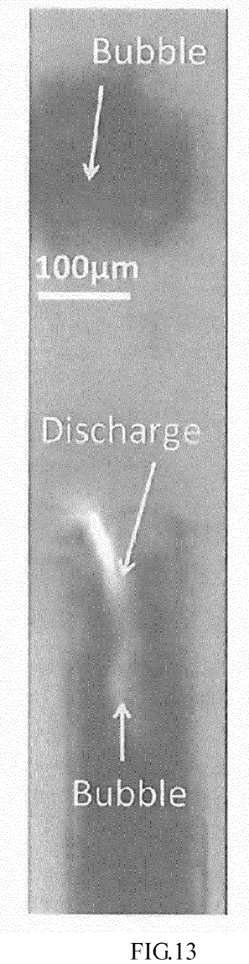

Bubbles were jetted in the same manner as in example 2, except that the solution used in the local ablation device and injection device prepared in example 2 was a 0.15M NaCl solution, the electricity output conditions were a current of 5.0 A and a voltage of 6.2 kV, the output frequency was 450 kHz, the sampling frequency for impedance matching was 450 kHz, and feedback was performed at 3.5 kHz. The formation of bubbles was captured using a VW 600 M (Keyence Corp.) at a frame rate of 230,000 fps. FIG. 13 is the captured photograph. As is clear from FIG. 13, in example 3, electric discharge from the electrode 31 was successfully confirmed in addition to bubble jetting. Also, because electric discharge was successfully confirmed, the bubbles presumably contained plasma in principle.

### [Key]

1, 1': Bubble-jetting chip, 2: Substrate, 3: Bubble-jetting portion, 4: Energizing portion, 5: Counter electrode, 6: Local ablation device, 7: Outer shell part, 8: Photoresist, 10: Processed object, 31: Electrode, 32: Shell part, 33: Extended section, 34: Space, 35: Bubble-jetting outlet, 36: Bubble, 37: Insulating layer, 41: Frame, 42: Solution, 51: Cap, 52: Support, 53: Movable frame, 54: Urging means, 56: Variable frame, 61: General commercial AC power supply device, 62: Electric wire, 63: Non-dielectric resistor, 64: Voltage amplification circuit, 71: Channel

## Claims

1. A bubble-jetting chip, comprising:
at least a substrate, an energizing portion, and a bubble-jetting portion;
the energizing portion being formed on the substrate;
the bubble-jetting portion comprising an electrode that is formed of a conductive material, a shell part that is formed of an insulating photosensitive resin, and an extended section that extends from the shell part, the shell part covering a periphery of the electrode, the extended section extending beyond a tip of the electrode, and the bubble-jetting portion further comprising a space formed between the extended section and the electrode; and
the electrode of the bubble-jetting portion being formed on the energizing portion.

2. The bubble-jetting chip according to claim 1, wherein two or more of the bubble-jetting portions are formed.

3. The bubble-jetting chip according to claim 2, wherein the heights of the bubble-jetting portions are different.

4. The bubble-jetting chip according to any of claims 1 to 3, wherein the photosensitive resin is a negative photoresist.

5. The bubble-jetting chip according to any of claims 1 to 4, wherein a counter electrode that constitutes an electrode pair with the electrode of the bubble-jetting portion is formed on the substrate.

6. The bubble-jetting chip according to any of claims 1 to 5, further comprising an outer shell part formed around the bubble-jetting portion, and a space between the bubble-jetting portion and the outer shell part being capable of storing a solution containing an injection material.

7. The bubble-jetting chip according to claim 6, wherein a channel for delivering a solution containing the injection material and/or a solution for forming an assisting flow is formed in the space.

8. A local ablation device, comprising the bubble-jetting chip according to any of claims 1 to 7.

9. An injection device, comprising the bubble-jetting chip according to any of claims 1 to 7.

10. A local ablation method, wherein:
at least the space of the bubble-jetting chip of the local ablation device according to claim 8 is filled with a solution;
high-frequency pulses are applied to an electrode pair configured with the electrode of the local ablation device and the counter electrode to cause bubbles to be released from the tip of the bubble-jetting portion; and
a processed object is processed with the bubbles.

11. The local ablation method according to claim 10, wherein electricity is discharged from the electrode when the high-frequency pulses are applied.

12. An injection method, wherein:
at least the space of the bubble-jetting chip of the injection device according to claim 9 is filled with a solution containing an injection material;
high-frequency pulses are applied to an electrode pair configured with the electrode of the injection device and the counter electrode to cause the release of bubbles onto which the solution containing the injection material is adsorbed; and
the injection material is introduced into the processed object while local ablation is performed on the processed object with the bubbles.

13. The injection method according to claim 12, wherein electricity is discharged from the electrode when the high-frequency pulses are applied.
